# EUROPEAN PATENT APPLICATION

(11) **EP 4 477 208 A1**
(43) Date of publication of application: **18.12.2024**
(21) Application number: 23749379.6
(22) Date of filing: 06.02.2023
(51) Int. Cl.: A61J 1/05, A61J 1/00

(54) **DEVICE FOR COLLECTING ISOLATED BIOLOGICAL SAMPLES**

(30) Priority: 07.02.2022 ES 202230192 U
(71) Applicant: Vitro, S.A., 41020 Sevilla (ES)
(72) Inventor: OLMO SEVILLA, Asunción, 41020 Sevilla (ES); ALVAREZ FERNÁNDEZ, María Stela, 41020 Sevilla (ES)
(74) Representative: Manuel Illescas y Asociados, S.L.
(86) International application number: PCT/ES2023/070060
(87) International publication number: WO 2023/148420

(57) **Abstract**

The present invention relates to a biological sample self-collection device that houses a collector (such as a "veil", buffer, swab, brush, etc.) for any biological material, preferably of vaginal origin. At the same time, it is designed to combine a reactive liquid with said substances for subsequent analysis thereof in a watertight manner. Its use includes the detection of pathogens, such as human papillomavirus, and the diagnosis of cervical cancer, among others.

## Description

### OBJECT OF THE INVENTION

The object of the invention falls within the technical field corresponding to medicine, specifically, in the sector related to devices for collecting isolated biological samples from a human subject.

The present invention relates to a device for self-collection of biological samples that contains a collector ("veil", buffer, swab, brush, etc.) of any biological material, preferably of vaginal origin; and that, at the same time, is capable of combining a reactive liquid with said substances for the subsequent analysis thereof in a sealed manner and its use for the detection of pathogens, such as human papillomavirus, and the diagnosis of cervical cancer, among others.

### BACKGROUND OF THE INVENTION

Devices for collecting biological samples from the vagina or the cervix for the detection of diseases related to the female reproductive system are known in the state of the art.

Collecting a biological sample such as blood, saliva, urine, etc., from a patient is the first step in many diagnostic procedures.

For the collection of biological samples from the woman's uterus, a substance collector (swab, brush, fabric, etc.) is used in a device or container with or without means of transport as a more agile method to subsequently analyse the sample in the laboratory.

In addition, in the state of the art there are devices in charge of housing the collector which has taken the biological sample and which comprise a chamber with a fluid that reacts with the collected substances. Said chamber can be broken by pressure or by tensile stress, releasing the reactive liquid.

However, the problem arises at the time of analysis of the sample taken. The devices existing in the state of the art are manipulated by a user, who extracts one of the parts that make up said device, increasing the risk of sample contamination, even if the environment in which it is performed is sterile, thus affecting the results and leading to errors in the diagnosis of diseases. As well as the risk of exposure to the biological sample and the means of transport, if carried, by the handler, with the consequent biological risk.

On the other hand, it can also lead to errors in taking the amount of liquid that has reacted with the sample, depending on the ability of the user in charge of performing the analysis, again, making it possible to detect conditions that may affect the result of the test.

### DESCRIPTION OF THE INVENTION

The present invention seeks to solve the proposed problems by incorporating a container designed to hold a biological sample collector, preferably of vaginal origin. Through screwing a cap on the container, where the cap contains a chamber enclosing a reactive liquid and is sealed at the bottom by a sheet that is broken when the aforementioned cap is screwed on, allowing the liquid released from the chamber to react inside the container with the biological samples collected in the collector.

The device of the invention allows the collection of biological samples isolated from a human subject, preferably from the vaginal cavities (among others), such as, cells, tissue or fluids and/or vaginal secretions, for the detection of pathogens, particularly human papillomavirus (HPV), and the diagnosis of uterine cancer. Examples of pathogens include, but are not limited to, in addition to human papillomavirus (HPV), Chlamydia trachomatis, Haemophilus ducreyi, Herpes simplex virus 1 and 2, Mycoplasma 30 genitalium, Mycoplasma hominis, Neisseria gonorrhoeae, Treponema pallidum, Trichomonas vaginalis, and Ureaplasma(urealyticum/parvum).

The device of the invention allows the self-collection of biological samples, without the need for specialized medical intervention.

In more detail, the device object of the invention comprises a container, as mentioned above, fitted with an essentially tubular body and open at the top with a collector inside that contains biological samples, preferably of vaginal origin, which have been previously collected. In addition, the container includes an upper head of diameter essentially wider than that of the tubular body where an internal thread is located.

On the other hand, the invention also comprises a cap with an upper section and with an external thread, said external thread being complementary to the internal thread of the head of the container to allow the upper coupling of the cap on said head of the container in a threaded manner.

The cap also includes a portion, which may be tubular and elongated, extending downward from the upper section of said cap to an area close to the bottom of the container, the tubular portion ending with a filter. Around the tubular portion, there is an annular reservoir that contains a reactive liquid and is sealed at the bottom by a breakable sheet.

The body of the container features a series of opening mechanisms, which may be pointed barbs. When the cap is screwed onto the container, these barbs pierce the lower sheet that encloses the reactive liquid, causing said liquid to flow through the body of the container and bathing the sample collector. The volume of liquid contained in the reservoir is sufficient to ensure that the amount of liquid that has reacted with the sample collected rises through the interior of the tubular portion through the filter, preventing larger particles from affecting the sample combined with the liquid.

To collect the combined sample that is in the tubular portion, the cap has another breakable sheet in its upper section, designed to facilitate the passage by breaking it of a pipette that is introduced below said tubular portion to reach the reactive liquid.

Finally, the device also comprises immobilization means that prevent the cap from unscrewing from the container once it is coupled, avoiding manipulation and contamination of the sample, remaining completely sealed until the moment of collection of the liquid by the pipette. Said immobilization means are a series of grooves of generally parallelepiped geometry located internally in the upper head of the container and external projections located in the upper section of the cap, which have an inclined surface that favours the rotational movement and subsequent engagement within the grooves.

Once they are inserted, the geometry of the projections prevents them from disengaging from the grooves, immobilizing the cap to the upper head of the container and, therefore, avoiding contamination and/or handling of the sample with particles from the environment.

Particularly, the device of the invention allows collecting biological samples from a human subject to diagnose and detect the presence of pathogens or diseases.

Thus, another aspect of the present invention is the in vitro use of the biological sample collection device of the invention, as described above for the detection of at least one pathogen.

In a particular embodiment, the pathogen detected by the in vitro use of the biological sample collection device of the invention is selected from the list consisting of: human papilloma virus (HPV), Chlamydia trachomatis, Haemophilus ducreyi, Herpes simplex virus 1 and 2, Mycoplasma genitalium, Mycoplasma hominis, Neisseria gonorrhoeae,Treponema pallidum, Trichomonas vaginalis and Ureaplasma(urealyticum/parvum), among others.

Further, another aspect of the present invention is the in vitro use of the biological sample collection device of the invention as described above for the diagnosis of uterine cancer.

### DESCRIPTION OF THE DRAWINGS

To complete the description being made and to aid in a better understanding of the characteristics of the invention, according to a preferred practical embodiment thereof, a set of drawings is included as an integral part of this description, wherein, in an illustrative and non-limiting manner, the following has been represented:
Figure 1.- It shows a perspective view of the biological sample collection device.
Figure 2.- It shows a section view of the biological sample collection device.

### PREFERRED EMBODIMENT OF THE INVENTION

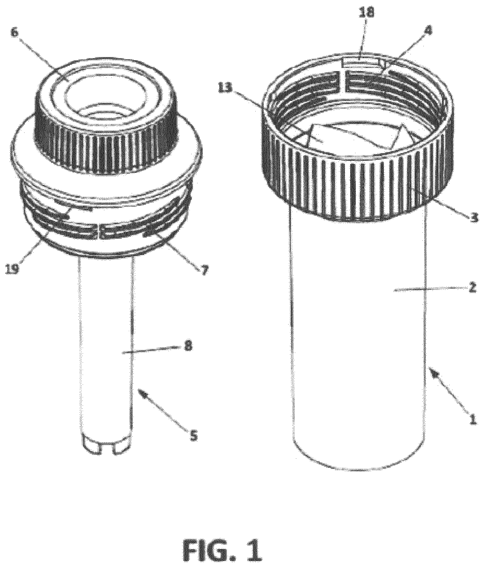

With the aid of Figures 1 to 2 an embodiment of a biological substance sampling device is described.

Figure 1 shows a perspective view of the biological sample collection device, which comprises a container (1) fitted with a lower open tubular body (2) intended to house a sample collection sheath (15) and an upper head (3) fitted with an internal thread (4); and a cap (5) fitted with an upper section (6) and incorporating an external thread (7), said cap (5) being designed to be screwed onto the internal thread (4) of the upper head (3) of the container (1). Additionally, the cap (5) includes a tubular portion (8) that extends downward from the upper section (6).

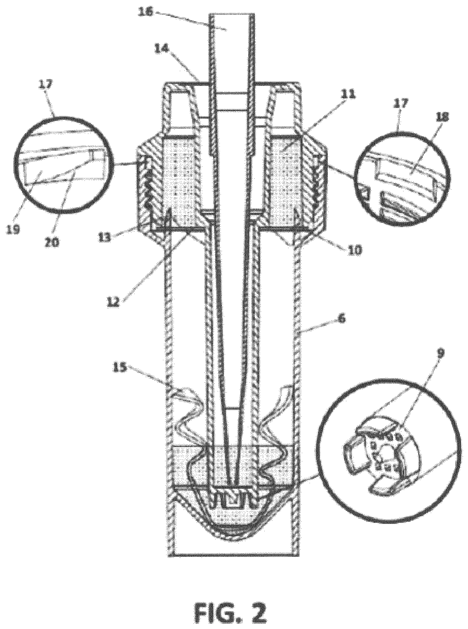

For a better visualization of the invention, Figure 2 shows a sectional view of the device presenting the cap (5) screwed onto the container (1) wherein the internal thread (4) and the external thread (7) may be complementary to each other.

The tubular portion (8), which extends downward, ends in a filter (9) at its tip. Surrounding this tubular portion (8), the cap (5) features an annular reservoir (10) that contains a reactive liquid (11). This annular reservoir (10) is sealed at the bottom by a breakable lower sheet (12). The tubular body (2) has barbs (13) that extend upward so that when the cap (5) is fitted onto the upper head (3) of the container (1), these barbs (13) break the lower sheet (12) allowing 25 the reactive liquid (11) to fall into the tubular body (2) and come into contact with the biological substances collected by the collector (1 5), reacting with them.

The volume of fluid in the annular reservoir (10) and the height of the tubular portion (8) are such that the reactive liquid (11) combined with the biological substances is partially introduced inside the tubular portion (8) through the filter (9).

On the other hand, the cap is characterized by also comprising a breakable upper sheet (14) that seals the passage to the tubular portion (11) and is designed to facilitate the entry of a pipette (16). This pipette is inserted through the tubular portion (8) to reach the reactive liquid (11) that has previously ascended, allowing it to take the biological sample from the combined biological sample collector (15) that has reacted with the reactive liquid (11) in a sealed manner, preventing any contamination of the sample.

Finally, to ensure the tightness of the sample and prevent, as mentioned, contamination of the sample and/or manipulation of the device by a user before the sample is analysed, the invention incorporates immobilization means (17) that secure the cap (5) once it is fitted onto the container (1). These immobilization means (17) consist essentially of prismatic grooves (18) located internally in the upper head (3) of the container (1); and projections (19) located in the upper section (6) of the cap (5) with an inclined surface (20) that facilitates the engagement of said projections (19) into the grooves (18) when the cap (15) is screwed onto the container (1). Once the projections (19) are engaged in the grooves (18), said surface (20) prevents them from being dislodged, thereby preventing the cap (3) from unscrewing from the container (1).

## Claims

1. A device for the collection and/or transport of isolated biological samples from a human subject, designed to house a biological sample collector (15), comprising: a container (1) with an open lower tubular body (2) intended to house the biological sample collector (15) and an upper head (3) with an internal thread (4); and a cap (5) with an upper section (6) incorporating an external thread (7), the cap (5) being intended to be screwed onto the internal thread (4) of the upper head (3) of the container (1); **characterised in that** the cap (5) additionally comprises a tubular portion (8) extending downward from the upper section (6) and ending in a filter (9), and an annular reservoir (10) located in the upper section (6) around the tubular portion (8) that contains a reactive liquid (11), the annular reservoir (10) being sealed at the bottom by a breakable lower sheet (12); and the tubular body (2) is equipped with barbs (13) extending upward such that when the cap (5) is screwed onto the upper head (3) of the container (1), these barbs (13) break the lower sheet (12), allowing the reactive liquid (11) to flow into the tubular body (2) and come into contact with the biological substances collected by the sample collector (15), reacting with them; the volume of fluid in the annular reservoir (10) and the height of the tubular portion (8) being such that the reactive liquid (11) combined with the biological substances is partially introduced through the tubular portion (8) via the filter (9); the cap is also **characterised by** comprising an additional breakable upper sheet (14) that closes off the tubular portion (11) and is designed to facilitate the insertion of a pipette (16) through the tubular portion (8) to reach the previously ascended reactive liquid (11); the device further includes immobilisation means (17) that secure the cap (5) once it is fitted onto the container (1), thus preventing manipulation and contamination of the collected sample.

2. Biological sample collection device according to claim 1 wherein the immobilization means (17) comprise:
- essentially prismatic grooves (18) that are located internally in the upper head (3) of the container (1); and
- projections (19) located in the upper section (6) of the cap (5) with an inclined surface (20) that facilitates the fitting of said projections (19) into the grooves (18) when the cap (15) is screwed onto the container (1), wherein, once the projections (19) are inserted into the grooves (18), said surface (20) prevents them from being dislodged and, consequently, prevents the unscrewing of the cap (3) from the container (1).

3. Biological sample collection device according to claim 1 wherein the internal thread (4) of the upper head (3) and the external thread (7) of the cap (5) are complementary.
